# EUROPEAN PATENT SPECIFICATION

(11) **EP 3 358 355 B1**
(45) Date of publication and mention of the grant of the patent: **11.10.2023**
(21) Application number: 17461508.8
(22) Date of filing: 04.02.2017
(51) Int. Cl.: G01N 33/68, G01N 33/564

(54) **USE OF SERUM 2-CYSTEINE PEROXIREDOXINS (2-CYS-PRDX) AS BIOMARKERS OF CHRONIC KIDNEY DISEASES (CKD) SUCH AS LUPUS NEPHRITIS (LN), IGA NEPHROPATHY (IGAN) AND AUTOSOMAL-DOMINANT POLYCYSTIC KIDNEY DISEASE (ADPKD) USEFUL FOR DIAGNOSING, THESE DISEASES AND METHOD OF DIFFERENTIATION OF THESE DISEASES**
VERWENDUNG VON SERUM 2-CYSTEIN PEROXIREDOXIN (2-CYS-PRDX) ALS BIOMARKER FÜR CHRONISCHE NIERENERKRANKUNGEN (CKD), WIE LUPUS NEPHRITIS (LN), IGA NEPHROPATHIE (IGAN) UND AUTOSOMAL-DOMINANTE POLYZYSTISCHE NIERENERKRANKUNG (ADPKD) ZUR DIAGNOSE DIESER ERKRANKUNGEN UND EIN VERFAHREN ZUR DIFFERENZIERUNG DIESER ERKRANKUNGEN
UTILISATION DES PÉROXYRÉDOXINES 2-CYSTÉINE (2-CYS-PRDX) SÉRIQUES COMME BIOMARQUEURS DE MALADIES RÉNALES CHRONIQUES (CKD) TELLES QUE LE LUPUS NEPHRITIS (LN), LA NÉPHROPATHIE À IGA (IGAN) ET LA POLYKYSTOSE RÉNALE TYPE DOMINANT (ADPKD) POUR LE DIAGNOSTIC DE CES MALADIES ET POUR LEUR DIFFÉRENCIATION

(43) Date of publication of application: 08.08.2018
(73) Proprietor: Warszawski Uniwersytet Medyczny, 02-091 Warszawa (PL)
(72) Inventor: MUCHA, Krzysztof, 01-651 Warszawa (PL); ZAGOZDZON, Radoslaw, 05-080 Truskaw (PL); FORONCEWICZ, Bartosz, 02-909 Warszawa (PL); PACZEK, Leszek, 03-603 Warszawa (PL); KRATA, Natalia, 35-513 Rzeszow (PL); GALA, Kamila, 01-923 Warszawa (PL)
(74) Representative: Patpol Kancelaria Patentowa Sp. z o.o.

(56) References cited:
- WO-A1-2005/116082
- AU-A1- 2015 202 230
- US-A1- 2014 038 203
- US-A1- 2014 221 325
- US-B2- 7 598 228
- MAGDALENA LUCZAK ET AL: "Label-Free Quantitative Proteomics Reveals Differences in Molecular Mechanism of Atherosclerosis Related and Non-Related to Chronic Kidney Disease", INTERNATIONAL JOURNAL OF MOLECULAR SCIENCES, vol. 17, no. 5, 2 May 2016 (2016-05-02), page 631, XP055379251, DOI: 10.3390/ijms17050631
- GRIET GLORIEUX ET AL: "New insights in molecular mechanisms involved in chronic kidney disease using high-resolution plasma proteome analysis", NEPHROLOGY DIALYSIS TRANSPLANTATION., vol. 30, no. 11, 9 July 2015 (2015-07-09), pages 1842-1852, XP055379411, GB ISSN: 0931-0509, DOI: 10.1093/ndt/gfv254
- E. El Eter ET AL: "Peroxiredoxin isoforms are associated with cardiovascular risk factors in type 2 diabetes mellitus", Brazilian Journal of Medical and Biological Research, vol. 48, no. 5, 1 May 2015 (2015-05-01), pages 465-469, XP055754423, ISSN: 0100-879X, DOI: 10.1590/1414-431x20144142

## Description

### TECHNICAL FIELD

The present invention relates to methods for diagnosis of lupus nephritis (LN) using serum 2-cysteine peroxiredoxins (2-Cys-PRDX or 2-Cys-prdxs) as biomarkers. Moreover, the present invention relates to a method of differentiation of of lupus nephritis (LN) from other chronic kidney disease (CKD) selected from the group consisting of IgA nephropathy (IgAN) and autosomal-dominant polycystic kidney disease (ADPKD) in a subject. Corresponding diagnostic kits are also provided herein.

### BACKGROUND OF THE INVENTION

Chronic kidney disease is a public health problem which, depending on a country, affects approximately 8-13% of population and this problem continues to grow (see, for example, Albers, B., "Prevalence of Chronic Kidney Disease varies a lot across Europe", European Renal Association - European Dialysis and Transplant Association, 2016, Vol. 4: 1-2 or Hill NR, Fatoba ST, Oke JL, Hirst JA, O'Callaghan CA, Lasserson DS, et al., "Global Prevalence of Chronic Kidney Disease-A Systematic Review and Meta-Analysis" PLoS ONE, 2016; 11(7)). For example, the prevalence of CKD is up to 14,2% in the USA, 10,2% in Norway, and 11,9% in Poland. It is estimated that over 4 million people in Poland suffer from CKD. The number of patients with end stage renal disease (ESRD) subjected to dialysis in Poland exceeds 20 000, in addition to 14 000 patients after kidney transplantation. It is known, that both, early stages of CKD as well as ESRD are associated with higher morbidity, mortality and increased healthcare utilization. Therefore, early detection of kidney dysfunction is essential for the proper treatment of CKD, prevention of its progression and subsequent damage to other organ. Based on the incidence, the main causes of CKD and ESRD include: diabetes mellitus, glomerulonephritis, cardiovascular diseases and ADPKD (see e.g, Vassalotti JA, Fox CH, Becker BN, "Risk factors and screening for chronic kidney disease", Adv. Chronic Kidney Dis., 2010, Vol. 17(3): 237-45 and ERA-EDTA Registry: ERA-EDTA Registry Annual Report 2014. Academic Medical Center, Department of Medical Informatics, Amsterdam, the Netherlands, 2016). However, the list of CKD causes is long and heterogenous. These diseases are of various origins and present distinct pathogenesis. For example, lupus nephritis is a classic autoimmune disease, whereas autosomal-dominant polycystic kidney disease is an inherited monogenetic disorder. IgA nephropathy is the most common primary glomerulonephritis worldwide leading to glomerular damage and in up to 30-40% to ESRD.

Generally, regardless of the underlying disease, CKD is characterized by comparable symptoms, but the progression to the ESRD depends on the primary disease as well as age, sex and the degree of renal insufficiency at diagnosis. Usually, initial diagnosis of CKD is based on such pathological symptoms, as erytrocyturia, hematuria, different degrees of proteinuria, decline in renal function, or any combination of the above. The patient also might present unspecific clinical symptoms like edema or hypertension. Unfortunately, none of the symptoms mentioned above are disease specific. Therefore, the final diagnosis usually requires histopathological evaluation based on the kidney biopsy. This is an invasive procedure that associates with significant risk (including death) and has several disadvantages (e.g. use of this method in long-term ambulatory follow-up is not possible), for both, the patients and clinicians (Chronic Kidney Disease Prognosis Consortium, Matsushita K, van der Velde M, Astor BC, Woodward M, Levey AS, de Jong PE, Coresh J, Gansevoort RT., "Association of estimated glomerular filtration rate and albuminuria with all-cause and cardiovascular mortality in general population cohorts: a collaborative meta-analysis", Lancet. 2010; Vol. 375: 2073-81). Due to risks involved, the need for new diagnostic methods that would be: a) less-invasive, b) repetitive, c) easy to use in ambulatory as well as in the clinical settings of CKD follow-up and enabling to differentiate between certain CKDs without renal biopsy, is increasingly growing (e.g. Mucha K. et al "How to diagnose and follow patients with glomerulonephritis without kidney biopsy?", Pol. Arch. Med. Wewn., 2016, Vol. 126 (7-8): 471-473).

There are numerous urine or serum components proposed as biomarkers of selected kidney diseases. Importantly, biopsy findings in several studies corroborated the applicability of such biomarkers (Watorek E, et al "IL-17A as a potential biomarker of IgA nephropathy", Pol Arch Med Wewn., 2015, Vol. 125: 204-206 and WO2012033999, "Biomarkers for predicting kidney and glomerular pathologies").

It is known, that progression of renal disorders is related to oxidative stress, understood as an imbalance between pro- and antioxidant systems. The main mediators of oxidative stress are reactive oxygen species (ROS) and their excessive concentrations may cause DNA, lipid or protein oxidation resulting in cellular damage. The toxic effects of ROS overproduction are counteracted by increasing antioxidant capacity of tissues, using natural antioxidants, such as catalase, glutathione-related enzymes or an enzymatic chain comprising peroxiredoxin-thioredoxin-thioredoxin reductase system (Modaresi A, Nafar M, Sahraei Z, "Oxidative stress in chronic kidney disease", Iran J Kidney Dis., 2015 May; 9(3):165-79).

One of such systems is represented by peroxiredoxin family (PRDX). PRDXs are 20-30 kDa proteins containing cysteine residue with antioxidant properties (Wood ZA, Schroder E, Robin HJ, Poole LB., "Structure, mechanism and regulation of peroxiredoxins", Trends Biochem. Sci., Vol. 2003, 28: 32-40). Mammalian cells express six isoforms of PRDXs (PRDX1-PRDX6) that are located in different cellular compartments. They are divided in three classes: typical 2-cysteine-PRDX (PRDX1-PRDX4), atypical 2-cysteine - PRDX (PRDX5) and 1-cysteine -PRDX (PRDX6) (Wood ZA, Schroder E, Robin HJ, Poole LB, "Structure, mechanism and regulation of peroxiredoxins", Trends Biochem. Sci., Vol. 2003, 28: 32-40). In general, they are a part of organism's immunodefense and redox-signaling systems, and they are also functioning as chaperones and regulators of signal transduction (Yang HY, Lee TH, "Antioxidant enzymes as redox-based biomarkers: a brief review", BMB Rep. 2015, Vol. 48(4): 200-208, and Jeong J, Kim Y, Kyung Seong J, Lee KJ, "Comprehensive identification of novel post-translational modifications in cellular peroxiredoxin 6", Proteomics, 2012, Vol. 12: 1452-1462). PRDXs possess the ability to reduce excessive levels of hydrogen peroxide, which is an example of reactive oxygen species (Atieh Modaresi, Mohsen Nafar, Zahra Sahraei., "Oxidative Stress in Chronic Kidney Disease.", Iranian Journal of Kidney Diseases. 2015, Vol. 9: 165-79).

Generally, oxidative stress can be considered as a disturbance in the natural ability of cells to keep the balance between pro- and antioxidant systems (Cachofeiro V, Goicochea M, de Vinuesa SG, Oubiña P, Lahera V, Luño J, "Oxidative stress and inflammation, a link between chronic kidney disease and cardiovascular disease", Kidney Int Suppl. 2008; Vol. (111): S4-9). ROS are generated through several enzymatic processes including cellular respiration (by the mitochondrial electron transport chain) or the activity of nicotinamide adenine dinucleotide phosphate (NADPH) oxidase enzyme complex. Several enzymes are responsible for removal of ROS, including superoxide dismutase (SOD), catalase, glutathione-related enzymes, peroxidase, the peroxide-redox-thioredoxin-thioredoxin reductase enzymatic chain, and a number of non-enzymatic antioxidants. While moderate levels of hydrogen peroxide are necessary for certain cellular processes, its excessive concentration may cause cell and tissue damage (Descamps-Latscha B, Drueke T, Witko-Sarsat V, "Dialysis induced oxidative stress: biological aspects, clinical consequences, and therapy" Semin Dial. 2001; Vol. 14: 193-9).

The contribution of the above mentioned factors in CKD development is not yet fully elucidated and their diagnostic utility is uncertain. However, many studies are aimed to define oxidative stress-related biomarkers that are able to diagnose CKD, and preferably also to differentiate between different CKD causes.

Karasawa et al. in "Autoantibodies to peroxiredoxin I and IV in patients with systemic autoimmune diseases" (Microbiol. Immunol., 2005, 49(1), 57-65) disclose the studies that promote understanding of the relationships between autoimmunity and oxidative stress, investigate whether autoimmunity to the anti-oxidative peroxiredoxin (PRDX) enzymes exists in patients with systemic autoimmune diseases. Among autoimmune disorders, an autoimmune kidney disease associated with systemic lupus erythematosus (SLE) is mentioned therein. However, no suggestion nor specific solution on how to differentiate SLE, and especially lupus nephritis (LN) from IgA nephropathy or ADPKD, is provided.

Furthermore, another publication of Karasawa et al., "Peroxiredoxin 2 is a novel autoantigen for anti-endothelial cell antibodies in systemic vasculitis" (Clin. Exp. Immunol., 2010, 161(3), 459-470) discloses identification of peroxiredoxin 2 (PRDX2) as an autoantigenic protein for AECA (anti-endothelial cell antibodies). Occurrence of anti-PRDX2 autoantibodies, measured by enzyme-linked immunosorbent assay (ELISA), was significantly higher in systemic vasculitis (60%) compared to those in collagen diseases without clinical vasculitis (7%, P < 0.01) and healthy controls (0%, P < 0.01). Taken together, these studies suggest that anti- PRDX2 autoantibodies would be a useful marker for systemic vasculitis and would be involved in the inflammatory processes of systemic vasculitis. Nevertheless, the authors did not mention any potential use of PRDX2 as useful biomarker for diagnosis, monitoring and prognosis of other renal diseases, especially lupus nephritis (LN). Also it is not mentioned or even suggested that this biomarker is a useful tool when used in combination with other members of peroxiredoxins family.

PRDX2 has also been identified as a protein characteristic of CKD and related to eGFR decline as shown by Luczak M. et al, "Label-Free Quantitative Proteomics Reveals Differences in Molecular Mechanism of Atherosclerosis Related and Non-Related to Chronic Kidney Disease", International Journal of Molecular Sciences, 17(5), 2 May 2016 631). The relative amount of PRDX2 in the plasma of CKD patients is increased together with the decline in the eGFR. The accumulation of PRDX2 was 2.03 times higher in the plasma of CKD3-4 patients (third and fourth stages of CKD) and 2.06 times higher in the plasma of CKD5 patients compared to the CKD1-2 group. Nevertheless, this publication is silent about use of PRDX2 in combination with other members of peroxiredoxins family. Moreover, the authors also did not mention any potential use of PRDX2 as useful biomarker for distinguishing between two different CKDs in stage 2 on average. Similarly, PRDX2 biomarker is also listed in US 2014/038203 among others markers useful for detection and/or prediction of the onset or magnitude of kidney diseases. Nevertheless acute kidney injuries (AKI) are named as the only kidney diseases. This publication does not disclose or even suggest the use of PRDX2 biomarker as serum biomarker, particularly useful in combination with other PRDX markers for distinguishing between at least two different CKDs.

In publication "Identification of autoantigens specific for systemic lupus erythematosus with central nervous system involvement" (Lupus, 2010, 19(6), 717-726) Iizuka et al. identified potential autoantigens specific for central nervous system (CNS) involvement in systemic lupus erythematosus (SLE), among which peroxiredoxin-4 (PRDX4) was listed. However, there is no mention about any other potential use of selected autoantigens, particularly no reference is made to any kidney disease. PRDX4 was, however, identified in US 2014/221325 as a serum and urine biomarker of a kidney disease. Nevertheless, it was mentioned as a biomarker for glomerulonephritis (GN) only. This patent publication further discloses a kit that is suitable for analysis of a kidney disease, which comprises, (a) a multi-well assay plate, (b) in one or more vials, containers, or compartments, a set of labelled detection antibodies specific for said human analytes; and (c) in one or more vials, containers, or compartments, a set of calibrator proteins. Yet, neither of these two publications suggest usefulness of PRDX4 as a biomarker for diagnosis, differentiating and/or assessment of the risk of chronic kidney disease (CKD). Finally, no kit that would be useful for simple differentiation between two CKDs was disclosed therein.

An autoantibody against peroxiredoxin-1 (IgG anti-PRDX1 antibody) was also identified in patients with systemic sclerosis in Iwata et al., "Autoantibody against peroxiredoxin I, an antioxidant enzyme, in patients with systemic sclerosis: possible association with oxidative stress" (Rheumatology, 2007, 46(5), 790-795). The presence of IgG anti-PRDX1 antibody was associated with longer disease duration, more frequent presence of pulmonary fibrosis, heart involvement, and anti-topoisomerase I antibody with increased levels of serum immunoglobulin and erythrocyte sedimentation rates. IgG anti-PRDX1 antibody levels also positively correlated with renal vascular damage and negatively with pulmonary function tests. However, this publication does not indicate any association between PRDX1 and systemic lupus erythematosus (SLE), especially lupus nephritis (LN).

EP 2 403 864 discloses polypeptides containing structural motifs associated with cell signalling and other biologically relevant activities, the compositions comprising such polypeptides, and methods of identifying and using the same. In a specific embodiment of this disclosure, the specific polypeptide is a human peroxiredoxin 5 isoform B polypeptide or an active fragment or variant thereof. In certain embodiments of discussed disclosure, the composition comprising such polypeptide may be used to treat or manage immunodeficiencies, including autoimmune diseases, disorders and conditions, such as, for example, glomerulonephritis such as IgA nephropathy. Nevertheless, proposed composition is not specifically directed to detection of the renal diseases and the entire disclosure is silent about differentiation of most important renal diseases.

Next patent publication, US 7 598 228, relates to the therapeutic methods and agents useful for treatment of diseases and disorders associated with decreased expression of AOP-1 gene or AOP-1 itself, such as renal diseases, for example lupus nephritis (LN). The AOP-1 is a protein that belongs to the peroxiredoxin family and in the literature is commonly called peroxiredoxin 3 (PRDX3). PRDX3 was found to be one of the agents potentially useful in treatment of diseases and disorders associated with its decreased levels. Document also refers to diagnostic kits comprising means for determining the level of AOP-1, for example by ELISA. However, the publication does not mention any potential use of PRDX3 as useful biomarker for diagnosis, monitoring and prognosis of other chronic kidney diseases (CKD), such as IgA nephropathy (IgAN) or autosomal-dominant polycystic kidney disease (ADPKD). Moreover, the kits as disclosed in this publication are not dedicated to diagnosis, monitoring and prognosis of these CKDs, and particularly, to distinguishing between two different CKDs.

US 8 883 754 relates to a method for inhibiting angiogenesis using peroxiredoxin II (PRDX2) inhibitor, and a method for preparing angiogenesis-inhibiting medicines using PRDX2 inhibitor. The diseases, ailments, and conditions to be prevented or treated by the composition disclosed therein and comprising PRDX2 inhibitor include autoimmune diseases, for example IgA nephropathy. None of other important renal diseases is mentioned therein.

US 8 927 220 is focused on development of a protein that can be used not only for diagnosis of IgA nephropathy and thin-glomerular-basement-membrane (referred also as "TGBM") nephropathy, but also as a biomarker useful for diagnosis of serious cases thereof. More particularly, this publication discloses a biomarker protein that shows increased/decreased levels in urine of IgA nephropathy patients or TGBM nephropathy patients compared to those in urine of normal people, and a diagnostic kit using the biomarker protein, which can be used to diagnose IgA nephropathy and TGBM nephropathy early, and also to predict and determine the degree of progression of the disease in advance. Nevertheless this publication is also silent about even potential usefulness of this biomarkers for diagnosis and differentiation of other important renal diseases.

US 2009/0269772 provides systems, methods, and apparatus for searching of specific combination of compounds of therapeutic interest. In some embodiments disclosed therein, specific compound combinations are identified, which affect a specific phenotype corresponding to a disease state. The term "disease state" refers, according to this publication, to the presence or stage of disease in a biological specimen and/or a subject from which the biological specimen was obtained and, in specific embodiment disclosed therein, the disease stage includes auto-immune and immune diseases such as, for example, IgA nephropathy and lupus erythematosus. In the nonlimiting list of exemplary human transcription factors that may be used in the methods and systems disclosed therein peroxiredoxin family and its specific member peroxiredoxin 3 (PRDX3) is mentioned. However, no possibility of differentiation between auto-immune and immune-related diseases is even suggested.

US 2013/0040833 provides genetic profiles associated with biological conditions and methods of applying these profiles in evaluating the biological conditions. As such, in one aspect, this disclosure is directed to a profile of one or more RNA transcripts obtained from microvesicles, wherein such RNA transcripts constitutes peroxiredoxin PRDX6. Diseases or other medical conditions as described therein include, among others, IgA nephropathy. The publication is silent, however, about any other renal diseases.

WO 2005/116082 relates to a high molecular weight complex of 2-cysteine peroxiredoxins and its application in a diagnostic medicine for such disorders, as neurodegenerative disorders, Alzheimer's disease, Down's syndrome, breast cancer or lung cancer and similar. In this publication pharmaceutical composition for the prophylaxis and treatment of these diseases, as well as, transgenic animal or plant that is resistant to environmental stress or such diseases is discussed. Moreover, the publication discloses the finding that 2-cysteine peroxiredoxin proteins occur in various forms that determine different structure and molecular weight thereof, functioning as a peroxidase in a low molecular weight structural form and as a molecular chaperone in a high molecular weight structural form. Nevertheless, even potential use of 2-cysteine peroxiredoxins as a renal disease biomarker has not been anticipated.

WO 2016/159960 discloses a system and process for performing diagnostic assays, and more particularly an automated immunoanalyzer system and process for performing diagnostic assays for infectious and autoimmune diseases wherein, among non-limiting examples of autoimmune disorders that can be diagnosed using the method disclosed herein, lupus nephritis is mentioned. Non-limiting examples of autoantigens include the member of peroxiredoxin family, peroxiredoxin 1. However, also this publication is silent about potential use of any marker, that would be useful for diagnosis and differentiation of the most important renal diseases, which, as a diseases of various types and origins, require diversified methods of treatment.

In summary, even though a number of different markers related to some renal diseases is significant, there still exists a need for providing highly selective and sensitive diagnostic methods and tests, which would enable diagnosis, differentiation and monitoring of the three most common and important chronic kidney diseases (CKD), namely: IgA nephropathy (IgAN), lupus nephritis (LN) and autosomal-dominant polycystic kidney disease (ADPKD), especially in their early stages, when the chance for recovery is greater. Moreover, there is a need for methods that are suitable to differentiate these three diseases, as well other forms of CKD. While ADPKD is a foreseeable progressive genetically-predisposed disease, in most cases the clinical course of IgAN and LN cannot be predicted at the time of diagnosis, as well as during first years of observation. Since both IgAN and LN are prominent causes of CKD, there is a pressing need for identifying prognostic or predictive biomarkers to aid the decision-making process in management of these diseases, especially in their early stages. The present invention allows to reduce the cost of a single diagnose in comparison with the expensive ultrasonography in combination with genetic diagnostics (identification of mutations in at least two genes: PKD1 and PKD2) of ADPKD, and biopsy in case of LN and IgAN, which also does not provide the unequivocal diagnosis. Moreover, the latter diseases may overlap.

The present invention addresses these needs and offers further advantages.

### DISCLOSURE OF INVENTION

The present invention aims to solve the above identified problems. The present invention relates to 2-Cys-PRDXs that are detected at differential serum protein concentrations in serum samples collected from patients suffering from lupus nephritis LN as compared to healthy controls, but also enables to differentiate LN patients from others, suffering from renal diseases such as IgAN or ADPKD, preferably at the earliest disease stages, when hematuria or proteinuria occurs. The scope of the invention is defined by the appended claims. Embodiments and descriptions no longer falling under the scope of the appended claims are considered merely as examples suitable for understanding the invention.

The inventors unexpectedly ascertained that all members of peroxiredoxin family, 2-Cys-PRDXs, can be used not only as prognostic or predictive biomarkers in LN, but also for differentiating patients initially diagnosed with CKD. Therefore, according to the present invention, the biomarkers such as 2-Cys-PRDXs may be used in non-invasive diagnostics of renal diseases, with special regard to lupus nephritis (LN), which besides its applicability for the diagnostic process, may prospectively lead to the explanation of their contribution to the pathophysiology of renal disorders.

### Definitions

Unless defined otherwise, all technical and/or scientific terms used herein have the same meaning as commonly understood by one of ordinary skill in the art to which this invention belongs. The terminology used in the present description is used for the purpose of describing particular embodiments only, and is not intended to limit the scope of the present invention.

The term "diagnosis" refers to any method by which the person skilled in the art can estimate and/or determine the presence or absence of a particular disease or condition (such as LN) in a subject. The term "diagnosis" does not mean that there is possibility to determine the presence or absence of a particular disease with 100% accuracy. Instead, the person skilled in the art will understand that the term "diagnosis" refers to an increased probability that a certain disease is present in the subject. According to the invention, the probability should be greater than, at least, 70% certainty, preferably greater than 80% certainty, more preferably greater than 90% certainty, most preferably greater than 99% depending on the specific marker(s) and combination thereof. The skilled person in the art often makes a diagnosis on the basis of one or more diagnostic indicators, i.e., a marker.

The term "differentiation" means distinguishing one specific chronic kidney disease from other chronic kidney disease with at least 70% certainty, preferably with 80% certainty, more preferably with at least 90% certainty, most preferably with at least 99% certainty. For example, when one or more prognostic indicators reach a sufficiently high level in samples obtained from the subject, it may signal that the patient exhibits an increased probability of experiencing specific disease such as LN, but not other CKD.

Term "marker" refers to the indicator, it's presence or it's specific amount in a sample obtained from the subject signals a probability that a given course or outcome will occur. For example, when one or more prognostic indicators reach a sufficiently high level in the samples obtained from the subjects, such level may signal that the patient exhibits an increased probability of experiencing specific disease such as LN in comparison to a similar patient exhibiting a lower marker level.

The term "biomarker" refers to the marker of biological origin.

The term "subject" refers to a human or non-human organism. Preferably, the subjects are "patients", i.e., living humans that are under medical care. This includes both, patients with defined and patients without any defined disease, who are being diagnosed.

The term "healthy control" refers to a human or non-human organism who is not suffering from any disease, or at least is not suffering from any renal disease.

The term "at least two of 2-Cys-PRDXs selected from a group consisting of PRDX1, PRDX2, PRDX3, PRDX4 and PRDX5" as used herein means 2, 3, 4 or 5 of listed group elements. In specific embodiments of the invention, the selected biomarkers include any two, any three, any four or five elements selected from PRDX1, PRDX2, PRDX3, PRDX4 and PRDX5.

The term "reference amount" as used herein is the amount of a biomarker as detected in a sample obtained from a subject without any condition/disease being assessed (i.e. from a healthy control). The reference amount is determined for each biomarker, as an average level or median level exhibited in a clinical population that does not exhibit the condition or disease to be detected. In potential methods for monitoring disease, the reference level can be biomarker's prior level of the subject.

The term "control" as used herein means the mechanisms that eliminate extraneous factors that might otherwise affect the results of a measurement. By creating another set, which is unaffected by the disease being assessed, one can isolate the disease by comparing the test group with the control group. For example, LN group is compared with IgAN, ADPKD or "healthy controls".

The term "corresponding" as used herein refers to directly the same biomarkers selected from PRDX1, PRDX2, PRDX3, PRDX4 and PRDX5 that are identified in a serum sample from the subject and in a serum sample from a healthy control. For example, if combination of PRDX1 and PRDX4 is identified the serum sample from the subject the corresponding combination of markers that is identified in a serum sample from a healthy control is the same combination of biomarkers - PRDX1 and PRDX4.

The term "proteinuria" as used herein is the urinary protein loss, measured in 24 hours urine collection. Such method of proteinuria evaluation has the best available accuracy.

The term "eGFR" as used herein is the estimated glomerular filtration rate, calculated with the use of Chronic Kidney Disease Epidemiology Collaboration (CKD-EPI) equation. Such method of estimating renal function is most suitable for CKD patients.

Additionally, the words such as: "comprise," "comprising," "include," "including," and "includes" when used in this specification and in the following claims are intended to specify the presence of stated features, integers, components, ingredients, compounds, species, steps, acts, or groups etc., but they do not preclude the presence or addition of one or more other integers, components, ingredients, compounds, species or steps, acts, or groups.

### DETAILED DESCRIPTION OF INVENTION

Present invention relates to the use of at least two of serum 2-cysteine peroxiredoxins (2-Cys-PRDXs or 2-Cys-prdxs) selected from a group consisting of PRDX1, PRDX2, PRDX3, PRDX4 and PRDX5 as serum biomarkers to diagnose lupus nephritis (LN) and differentiate lupus nephritis (LN) from other chronic kidney disease (CKD) selected from the group consisting of IgA nephropathy (IgAN) and autosomal-dominant polycystic kidney disease (ADPKD). More preferably, at least two 2-Cys-PRDX biomarkers are selected from a combination of PRDX1+PRDX2, PRDX1+PRDX3, PRDX1+PRDX4, PRDX1+PRDX5, PRDX2+PRDX3, PRDX2+PRDX4, PRDX2+PRDX5, PRDX3+PRDX4, PRDX3+PRDX5 or PRDX4+PRDX5 and most preferably at least two 2-Cys-PRDX biomarkers are selected from a combination of PRDX1+PRDX4, PRDX2+PRDX4 or PRDX4+PRDX5. In even more preferable embodiment, at least three 2-Cys-PRDX biomarkers are used, which are selected from a combination of PRDX1+PRDX2+PRDX3, PRDX1+PRDX2+PRDX4, PRDX1+PRDX2+PRDX5, PRDX1+PRDX3+PRDX4, PRDX1+PRDX3+PRDX5, PRDX1+PRDX4+PRDX5, PRDX2+PRDX3+PRDX4, PRDX2+PRDX3+PRDX5, PRDX2+PRD$+PRDX5, or PRDX3+PRDX4+PRDX5. In other preferred embodiments of the invention at least four 2-Cys-PRDX biomarkers are used, which are selected from a combination of PRDX1+PRDX2+PRDX3+PRDX4, PRDX1+PRDX2+PRDX3+PRDX5, PRDX1+PRDX2+PRDX4+PRDX5, PRDX1+PRDX3+PRDX4+PRDX5, or PRDX2+PRDX3+PRDX4+PRDX5. In yet another preferred embodiment of the invention all five 2-Cys-PRDX biomarkers are used as a combination of PRDX1+ PRDX2+PRDX3+PRDX4+PRDX5.

Present invention also relates to the method of diagnosis of lupus nephritis (LN) and differentiation of lupus nephritis (LN) from other chronic kidney disease (CKD), selected from the group consisting of lupus nephritis (LN), IgA nephropathy (IgAN) and autosomal-dominant polycystic kidney disease (ADPKD) in a subject, comprising:
a) a step of identification of at least two, three, four or five 2-Cys-PRDX biomarkers selected from a group consisting of PRDX1, PRDX2, PRDX3, PRDX4 and PRDX5 in a serum sample from said subject, and
b) a step of identification of at least at least two or three or four or five biomarkers selected from a group consisting of PRDX1, PRDX2, PRDX3, PRDX4 and PRDX5 in a serum sample from a healthy subject, wherein said 2-Cys-PRDX biomarkers correspond to the biomarkers identified in step (a), and
c) a step of comparison of the 2-Cys-PRDX biomarkers identified in step (a) in a serum sample from said subject with the corresponding 2-Cys-PRDX biomarkers identified in step (b) in a serum sample from a healthy subject wherein elevated biomarkers concentrations in serum diagnose LN and differentiate LN from other CKD-related diseases selected from the group consisting of IgA nephropathy (IgAN) and autosomal dominant polycystic kidney disease (ADPKD).

Present disclosure also relates to a kit of ELISA tests, not being part of the invention, suitable for diagnosis and differentiation of chronic kidney disease (CKD) selected from lupus nephritis (LN), IgA nephropathy (IgAN) and/or autosomal-dominant polycystic kidney disease (ADPKD) in a subject, wherein each of the ELISA tests comprises a set of antibodies that specifically binds to a different 2-Cys-PRDX biomarkers of a CKD selected from a group consisting of PRDX1, PRDX2, PRDX3, PRDX4 and PRDX5. Preferably, antibody or antibodies are conjugated to a label. Even more preferably, the kit comprises a microfluidic chip.

The kit can be any kit suitable to use with biological fluid sample with such proviso, that it comprises antibodies that specifically bind specific CKD biomarkers of the present disclosure. In other words, the kit is configured to conduct a multiplexed assay of two or more of analytes such as CKD biomarkers of the present disclosure and comprise:
(a) a single panel arrayed on a multi-well plate which is configured to be used in an electrochemiluminescence assay, and
(b) associated consumables, e.g., detection antibodies, calibrators, and optional diluents and/or buffers.
Alternatively, the multi-well plates and associated consumables can be provided separately. A capture antibody to each analyte is immobilized on a binding domain in the well and that capture antibody is used to detect the presence of the target analyte in an immunoassay. Briefly, a sample suspected of containing that analyte is added to the well and if present, the analyte binds to the capture antibody at the designated binding domain. The presence of bound analyte on the binding domain is detected by adding labeled detection antibody. The detection antibody also binds to the analyte forming a "sandwich" complex (capture antibody-analyte-detection antibody) on the binding domain. The multiplexed immunoassay kits described herein allow a user to simultaneously quantify multiple biomarkers.

Despite of the fact that the kits comprising 2-Cys-PRDX biomarkers are known, the kit consisting of specific five 2-Cys-PRDX biomarkers only that is simple to use and based on fast, cheap and simple analysis of the biological sample provides fast diagnosis, enables differentiation of specific chronic kidney diseases (CKD), selected from lupus nephritis (LN), IgA nephropathy (IgAN) and/or autosomal-dominant polycystic kidney disease (ADPKD), which are characterized by comparable symptoms, have never before been reported. The present invention also relates to the use of 2-Cys-PRDX biomarkers selected from a group consisting of PRDX1, PRDX2, PRDX3, PRDX4 and PRDX5 for differentiation of chronic kidney disease (CKD) selected from the group consisting of lupus nephritis (LN), IgA nephropathy (IgAN) and autosomal-dominant polycystic kidney disease (ADPKD) in a subject.

All the inventions as claimed are described below in examples of practical realization thereof.

### BRIEF DESCRIPTION OF THE DRAWINGS

Present invention is described herein in relation to the following figures of drawings in which:

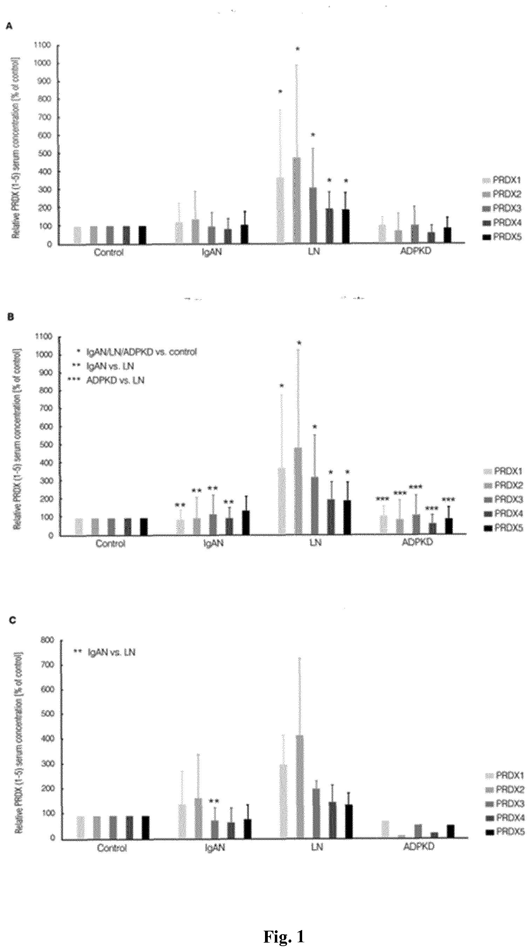

Fig. 1 presents 2-Cys-prdxs (1-5) serum concentration in patients diagnosed with IgAN, LN, ADPKD and healthy, age-, sex-matched control. Panel A (Fig. 1A) - reflects control group without any kidney disease or other chronic diseases requiring treatment, the remaining groups have CKD. Bars are representing the mean value of PRDX (1-5) serum concentration (the mark of PRDXs isoforms, changes with progressively darker shades of grey on a plot), the box itself is the standard error (SE) of the results; the whiskers are the upper standard deviation (SD). Panel B and C (Figs 1B and 1C, respectively) represent serum levels of 2-cys-prdxs in female (B) and male (C) patients separately, in comparison with healthy sex-matched control. Mann Whitney - U Tests were used to compare PRDX (1-5) concentration values of each group. Values with p<0.05 were considered statistically significant and marked on a graphs with: * - statistical significance of all groups comparing to control group; ** - IgAN patients comparing to LN patients; *** - ADPKD patients comparing to LN patients.

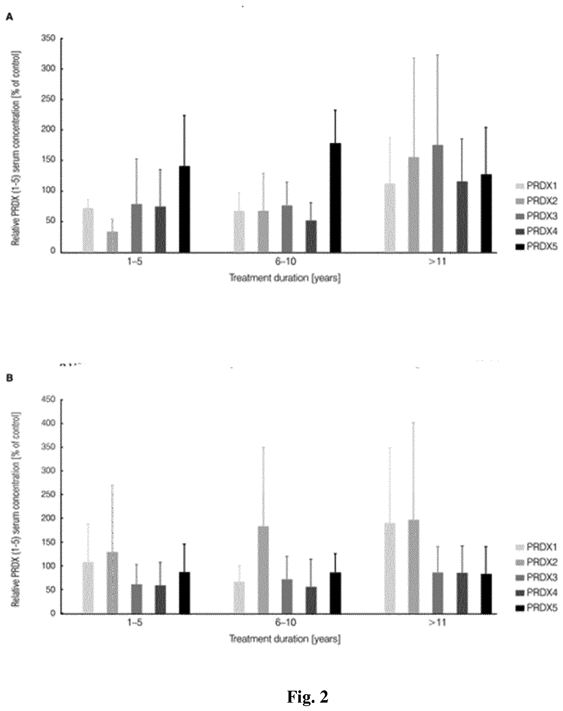

Fig. 2 presents 2-Cys-prdxs (1-5) serum concentration depending on the disease duration in female (A) and male (B) patients diagnosed with IgAN (Figs 2A and 2B respectively). Bars are representing mean value of PRDX (1-5) concentration, the box - standard error (SE), the whiskers are upper standard deviation (SD).

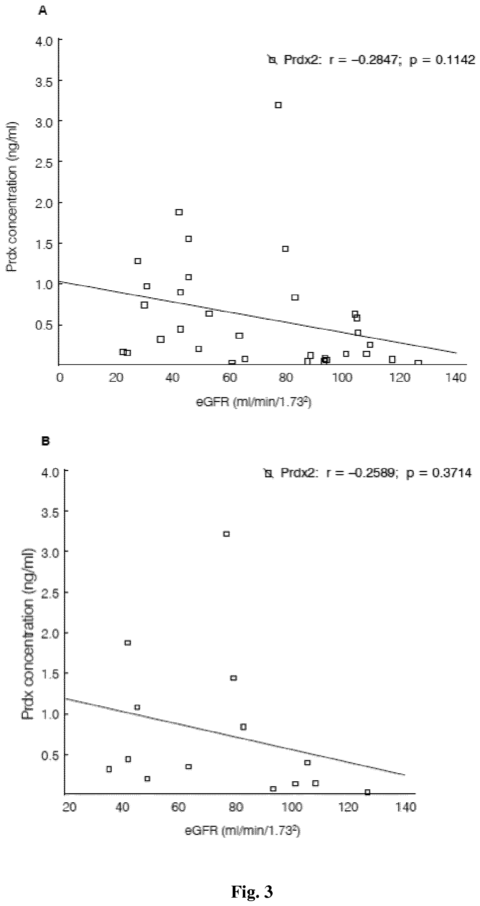

Fig. 3 is a graphical illustration of the correlation analysis of PRDX2 serum concentration and eGFR (calculated according to CKD-EPI equation) in patients diagnosed with IgAN. Panel A - reflects both male and female, B - only female, C - only male IgAN patients. The line parameters such as: r - Pearsons coefficient, p - statistical significance (p<0.05 are statistical significant) are also presented on a graph.

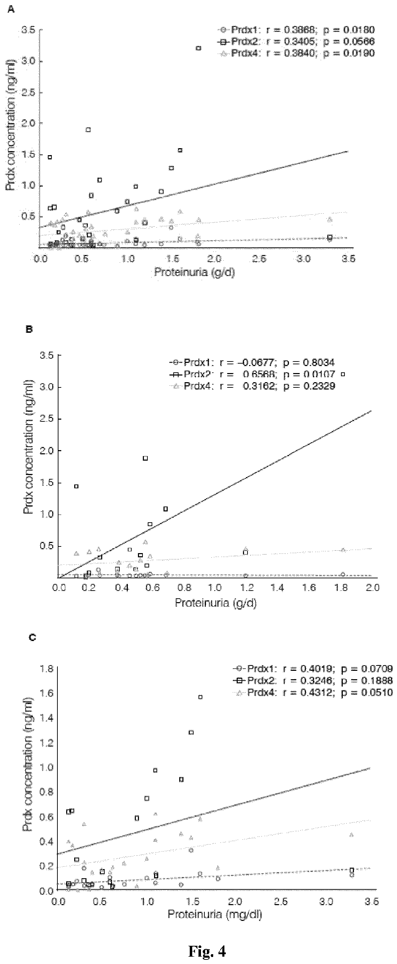

Fig. 4 is a graphical illustration of the correlation analysis of PRDX (1, 2 or 4) serum concentration and proteinuria in both male and female (Fig. 4A), only female (Fig. 4B) and only male (Fig. 4C) patients diagnosed with IgAN. Three independent lines are presented, with parameters such as: r - Pearsons coefficient, p - statistical significance (p<0.05 are statistical significant) are presented on a graph.

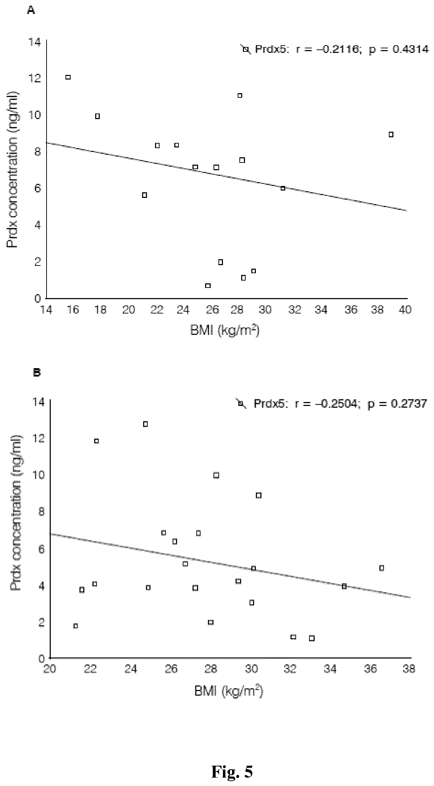

Fig. 5 is a graphical illustration of the correlation analysis of PRDX5 serum concentration and BMI in female (Fig. 5A), male (Fig. 5B) patients diagnosed with IgAN and in female patients diagnosed with LN (Fig. 5C). Parameters such as: r - Pearsons coefficient and p - statistical significance (p<0.05 are statistical significant) are presented on a graph.

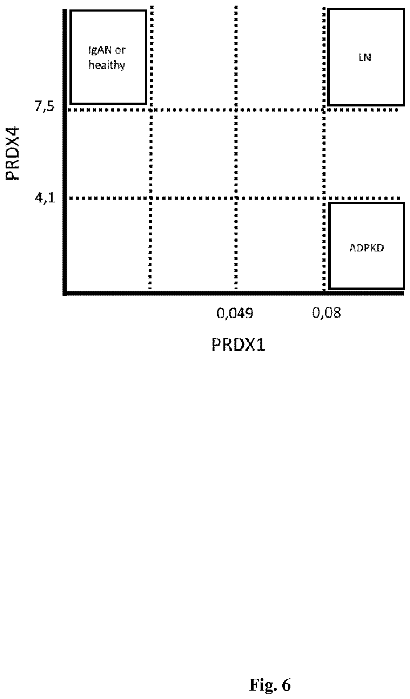

Fig. 6 is a schematic scheme of the differentiation of LN, ADPKD, IgAN in the correlation with the PRDX (1 and 4) titre. Additional statistical analysis indicates that parameter PRDX1 should help to distinguish patient from a healthy control, as well as to distinguish a patient with LN and with ADPKD from a patient with IgAN (PRDX1>0,08). Moreover, exclusion of ADPKD disease occurs when parameter PRDX4 exceeds value 7,5.

### EXAMPLES

### Participants

This study was performed using serum samples collected from 38 patients with biopsy-proven IgAN, 18 patients with biopsy-proven LN, 8 patients diagnosed with ADPKD and 15 healthy volunteers serving as the age- and sex-matched controls. The inclusion criteria for the control group were as follows: age older than 18 years and absence of any kidney disease or other chronic diseases requiring treatment. The exclusion criteria for both groups included: active infection, history of malignancy, previous organ transplantation, or current pregnancy. The blood was drawn once, at different stages of disease activity and duration. The study protocol was approved by the local ethics committee and informed consent was obtained from all participants. The study was performed in accordance with the Declaration of Helsinki. Demographic and clinical data of all groups is presented in Table 1 (values are given as mean ± SD)

**Table 1**

| **Charecteristics** | **IgA (n=38)** | **SLE (n=17)** | **ADPKD (n=7)** | **Control (n=15)** |
|---|---|---|---|---|
| **Male/ female (n)** | 21/17 | 2/15 | 1/6 | 6/9 |
| **Age (years)** | 40.37 ± 13.70 | 43.94 ± 12.37 | 51.86± 6.20 | 38.20 ± 9.79 |
| **BMI (kg/m²)** | 27.03 ± 4.85 | 23.43 ± 3.86 | 26.65 ± 6.17 | 24.34 ± 3.39 |
| **Serum creatinine (mg/dl)** | 1.31 ± 0.69 | 0.99 ± 0.46 | 1.34 ± 0.63 | 0.83 ± 0.15 |
| **eGFR (ml/min/1.73²)** | 74.70 ± 32.86 | 80.96 ± 26.10 | 57.05 ± 22.70 | 101.73 ± 14.70 |
| **Proteinuria (g/d)** | 0.73 ± 0.65 | 0.40 ± 0.60 | none | none |
| **HCT (%)** | 42.85 ± 4.11 | 39.64 ± 4.06 | 43.69 ± 1.43 | 43.76 ± 3.25 |
| **HGB (g/dl)** | 14.27 ± 1.50 | 12.78 ± 1.49 | 14.57 ± 0.75 | 14.71 ± 1.17 |

### Sample measurements

The serum concentration of each of the 2-Cys-PRDXs (1-5) was assessed in five independent ELISA tests (EIAab, Wuhan, China). Tests were proceeded strictly according to manufacturer's instructions.

In brief summary, the microtiter plate provided with ELISA kit has been precoated with an antibody specific to target antigen. The standards and samples were added in a predetermined order in amount of 100 µ1 to the appropriate wells with a biotin-conjugated antibody preparation, specific to target antigen, then avidin conjugated to horseradish peroxidase were added to each microplate well. The enzyme-substrate reaction was terminated by the addition of a sulphuric acid solution. Changes of colour in each well were measured spectrophotometrically at the wavelength of 450 nm. The concentration of targeted antigen in the samples were determined by comparing the O.D. of the samples to the standard curve.

### Statistical Analysis

The measurements of 80 samples in each test were collected, divided into appropriate groups (depending on type of disease), expressed as means ± SD and statistically analysed (Statistica 12.0, StatSoft^{®}). The statistical analysis comparing LN, IgAN and ADPKD patients to healthy controls were performed using Mann Whitney U Test (where p<0.05 was considered significant).

### Results discussion

If not provided otherwise, when a titre of concentration of PRDX is mentioned it should be understood as ng/ml.

As presented in Fig. 1A, the mean concentrations of PRDX1-5 in overall population of IgAN and ADPKD patients were not significantly different from the controls. In comparison, serum concentrations of all PRDXs were significantly elevated in LN patients, as compared to the control group. Notably, there was a robust intragroup variability in this group, especially in regard to PRDX1 and 2 concentrations, which corresponds to the complex nature of LN as a disease. This results prove that PRDX1-5 are selective biomarkers of LN.

Moreover, further statistical analysis for IgAN, LN, ADPKD and control, grouped by sex, revealed that more significant differences can be observed in the female (Fig. 1B) than in the male (Fig. 1C) population, which suggests that the oxidative stress management may vary between genders. PRDX 2 serum concentration is significantly correlated to proteinuria in females with IgAN, but not in LN or ADPKD. This fact may be used in differentiation of IgAN from the latter diseases during pregnancy, when renal biopsy is highly unrecommended. It suggests that comparison of serum concentration of at least two of five (1-5) isoforms of PRDXs might be also used as a preliminary tool for CKD diagnosis.

Furthermore, statistical analysis also shows that PRDX3 (PRDX3>5,03) is an important parameter which is influencing the LN prognosis. Change of PRDX3 parameter by value of 1ng/ml increases the LN probability by 12,401% (Pr>0,00879).

As can be seen from Fig. 2 disease duration in IgAN patients has no significant influence on the PRDX 1-5 serum concentrations. Therefore, PRDX 1-5 cannot be used to diagnose and differentiate IgAN from other CKD disease at any time from disease's onset. Differently, disease duration in LN patients has a significant influence on the PRDX 1-5 serum concentrations. Therefore, PRDX 1-5 can be used to diagnose and differentiate LN from other CKD disease at any time from disease's onset.

As can be seen from Fig. 3 the eGFR is inversely correlated with renal function. PRDX 1-5 serum concentrations in IgAN patients have no significant correlation to the eGFR. Therefore, PRDX 1-5 can be used to diagnose and differentiate IgAN from other CKD at any stage of renal impairment. PRDX 1-5 serum concentrations in LN patients have no significant correlation to the eGFR. Therefore, PRDX 1-5 can be used to diagnose and differentiate LN from other CKD at any stage of renal impairment.

As is evident from Fig. 4 proteinuria depends on the activity of glomerular diseases. PRDX 1 and 4 serum concentrations have significant correlation to the daily urinary protein loss in IgAN patients. Therefore, PRDX 1 and 4 may be used to assess disease activity. PRDX 2 serum concentration is significantly correlated to proteinuria in females with IgAN. Therefore, PRDX 2 may be used to assess disease activity in this subgroup of IgAN patients. Neither PRDX 1, nor 2, nor 4 serum concentrations in males with IgAN correlate significantly to proteinuria. Therefore, PRDX 1, 2 and 4 in males serum can be used to differentiate IgAN from other CKD regardless of the degree of proteinuria.

PRDX 1-5 serum concentrations have no significant correlations to the daily urinary protein loss in LN patients. Therefore, PRDX 1-5 can be used to diagnose and differentiate LN from other CKD regardless of the degree of proteinuria.

Since BMI may be correlated with proteinuria and is associated with eGFR, as it can be seen on Fig. 5, PRDX 1-5 serum concentrations have no significant correlations to the BMI in IgAN patients. Therefore, PRDX 1-5 can be used to differentiate IgAN from other CKD regardless of the body weight. On the contrary PRDX 1-4 serum concentrations have no significant correlations to the BMI in LN patients. Therefore, PRDX 1-4 can be used to differentiate LN from other CKD regardless of the body weight.

Moreover, PRDX 5 serum concentration is significantly correlated to the BMI in LN female patients (Fig. 5C). Therefore, PRDX 5 concentration should be interpreted with caution in obese females.

As can be seen from Fig. 6 combination of two 2-Cys-PRDXs is useful to differentiate chronic kidney disease (CKD) selected from the group comprising lupus nephritis (LN), IgA nephropathy (IgAN) and autosomal-dominant polycystic kidney disease (ADPKD). In case of combination of PRDX-1 and PRDX4 the titer of PRDX1 greater than 0,08 suggest that the subject does not suffer from IgA. To differentiate unequivocally patients suffering from LN and ADPKD it is sufficient to determine titer of PRDX4.

Additionally, based on the results as obtained and presented on the Figures the following can/might be concluded:
1. PRDX1 or PRDX2 distinguishes between healthy controls and patients with CKD;
2. PRDX2 or PRDX3 distinguishes IgAN from all subjects. Moreover, every PRDX distinguishes IgA from LN, whereas PRDX4 distinguishes IgAN from ADPKD.
3. PRDX 2 or PRDX3 or PRDX4 or PRDX5 distinguishes LN from all subjects, as well as from IgA and from ADPKD. PRDX 1 or PRDX2 or PRDX3 or PRDX4 or PRDX5 distinguishes LN from IgA and from ADPKD.

Summarized parameters that differentiate between all three diseases are shown in the Table 2 below:

**Table 2**

| | All | Control | IgAN | LN | ADPKD |
|---|---|---|---|---|---|
| All | X | 1,2 | 2,3 | 2,3,4,5 | 1,4 |
| Control | | X | none | 1,2,3,4,5 | 4 |
| IgAN | | | X | 1,2,3,4,5 | 4 |
| LN | | | | X | 1,2,3,4,5 |
| ADPKD | | | | | X |

### Results interpretation

The present study showed that serum levels of all 2-Cys-PRDXs were elevated in LN patients, while in other diseases (IgAN and ADPKD) they were on similar levels as in healthy controls (Fig. 1A). Indeed, the statistical analysis using Mann Whitney U Test, revealed that there were no statistically significant differences between healthy controls and patients with IgAN or ADPKD. However, there was a difference in each PRDXs isoform (1-5) serum concentration in patients with LN (p<0.05).

Therefore, it was claimed that elevated 2-Cys-PRDXs concentrations in serum could be a useful biomarker of LN versus other CKD-related diseases, in addition to currently used markers such as a range of autoantibodies and other markers in LN. Moreover, PRDX 1-5 can be used to diagnose and differentiate LN from other CKD at any time from disease's onset, at any stage of renal impairment, and regardless of the degree of proteinuria or BMI (except for PRDX 5 in females).Additionally, correlation between PRDX2 serum concentration and estimated glomerular filtration rate (eGFR), which was calculated according to CKD-EPI equation in patients diagnosed with IgAN (Fig. 4), correlation between PRDX (1, 2 or 4) serum concentration and proteinuria (Fig. 5) and correlation between PRDX5 serum concentration and BMI (Fig. 6) shows the association of these parameters with the 2-Cys-PRDXs serum levels. As presented on the Figures 4-6 such correlation might also be used to differentiate patients with various types or mechanisms of kidney failure and improve early-diagnosis methods. Therefore, the studies summarized above prove that serum concentrations of 2-Cys-PRDXs are very promising new biomarkers, which can improve current diagnostic methods of CKD and are sufficiently sensitive and accurate to detect early LN and/or to monitor its progression.

It must be underlined that the present invention is both cost-efficient, as well as, hassle-free for the potential subject, or a patient since it doesn't require use of any equipment such as USG or distress due to a biopsy which are perceived as onerous and harmful for health, especially in pregnant patients.

## Claims

1. Use of at least two of serum 2-Cys-PRDXs selected from a group consisting of PRDX1, PRDX2, PRDX3, PRDX4 and PRDX5 as a serum biomarker to diagnose lupus nephritis (LN) and differentiate lupus nephritis (LN) from other chronic kidney diseases (CKD) selected from the group consisting of IgA nephropathy (IgAN) and autosomal dominant polycystic kidney disease (ADPKD), wherein, when the at least two 2-Cys-PRDX biomarkers comprise a combination of PRDX1+PRDX4 lupus nephritis (LN), IgA nephropathy (IgAN) and autosomal-dominant polycystic kidney disease (ADPKD) can be further differentiated.

2. The use according to the claim 1, wherein at least two 2-Cys-PRDXs biomarkers are selected from a combination of PRDX1+PRDX2, PRDX1+PRDX3, PRDX1+PRDX4, PRDX1+PRDX5, PRDX2+PRDX3, PRDX2+PRDX4, PRDX2+PRDX5, PRDX3+PRDX4, PRDX3+PRDX5, or PRDX4+PRDX5.

3. The use according to claim 1 or 2, wherein at least three 2-Cys-PRDXs biomarkers are used, which are selected from a combination of PRDX1+PRDX2+PRDX3, PRDX1+PRDX2+PRDX4, PRDX1+PRDX2+PRDX5, PRDX1+PRDX3+PRDX4, PRDX1+PRDX3+PRDX5, PRDX1+PRDX4+PRDX5, PRDX2+PRDX3+PRDX4, PRDX2+PRDX3+PRDX5, PRDX2+PRD4+PRDX5, or PRDX3+PRDX4+PRDX5.

4. The use according to any of claims 1-3 wherein at least four 2-Cys-PRDXs biomarkers are used, which are selected from a combination of PRDX1+PRDX2+PRDX3+PRDX4, PRDX1+PRDX2+PRDX3+PRDX5, PRDX1+PRDX2+PRDX4+PRDX5, PRDX1+PRDX3+PRDX4+PRDX5, or PRDX2+PRDX3+PRDX4+PRDX5.

5. The use according to any of claims 1-4, wherein all five 2-Cys-PRDXs biomarkers PRDX1+ PRDX2+PRDX3+PRDX4+PRDX5 are used.

6. A method of diagnosis of lupus nephritis (LN) and differentiation of lupus nephritis (LN) from other chronic kidney disease (CKD) selected from the group consisting of IgA nephropathy (IgAN) and autosomal-dominant polycystic kidney disease (ADPKD) in a subject, comprising
(a) a step of identification of at least two, three, four or five 2-Cys-PRDXs biomarkers selected from a group consisting of PRDX1, PRDX2, PRDX3, PRDX4 and PRDX5 in a serum sample from said subject, and
(b) a step of identification of at least two or three or four or five biomarkers selected from a group consisting of PRDX1, PRDX2, PRDX3, PRDX4 and PRDX5 in a serum sample from healthy subject, and
(c) a step of comparison of identified biomarkers in a serum sample from said subject with the markers identified in a serum sample from healthy subject wherein elevated biomarkers concentrations in serum diagnose LN and differentiate LN from other CKD-related diseases selected from the group consisting of IgA nephropathy (IgAN) and autosomal dominant polycystic kidney disease (ADPKD).

## Patentansprüche

1. Verwendung von mindestens zwei Seren 2-Cys-PRDXs, ausgewählt aus einer Gruppe, die aus PRDX1, PRDX2, PRDX3, PRDX4 und PRDX5 besteht, als Serum-Biomarker zur Diagnose von Lupus-Nephritis (LN) und zur Unterscheidung der Lupus-Nephritis (LN) von anderen chronischen Nierenerkrankungen (CKD), ausgewählt aus der Gruppe, die aus IgA-Nephropathie (IgAN) und autosomal-dominanter polyzystischer Nierenerkrankung (ADPKD) besteht, wobei, wenn mindestens zwei 2-Cys-PRDX-Biomarker eine Kombination von PRDX1+PRDX4 enthalten, Lupus-Nephritis (LN), IgA-Nephropathie (IgAN) und autosomal-dominante polyzystische Nierenerkrankung (ADPKD) weiter differenziert werden können.

2. Verwendung nach Anspruch 1, wobei mindestens zwei 2-Cys-PRDXs-Biomarker aus einer Kombination von PRDX1+PRDX2, PRDX1+PRDX3, PRDX1+PRDX4, PRDX1+PRDX5, PRDX2+PRDX3, PRDX2+PRDX4, PRDX2+PRDX5, PRDX3+PRDX4, PRDX3+PRDX5 oder PRDX4+PRDX5 ausgewählt sind.

3. Verwendung nach Anspruch 1 oder 2, wobei mindestens drei 2-Cys-PRDXs-Biomarker verwendet werden, die aus einer Kombination von PRDX1+PRDX2+PRDX3, PRDX1+PRDX2+PRDX4, PRDX1+PRDX2+PRDX5, PRDX1+PRDX3+PRDX4, PRDX1+PRDX3+PRDX5, PRDX1+PRDX4+PRDX5, PRDX2+PRDX3+PRDX4, PRDX2+PRDX3+PRDX5, PRDX2+PRD4+PRDX5 oder PRDX3+PRDX4+PRDX5 ausgewählt sind.

4. Verwendung nach einem der Ansprüche 1-3, wobei mindestens vier 2-Cys-PRDXs-Biomarker verwendet werden, die aus einer Kombination von PRDX1+PRDX2+PRDX3+PRDX4, PRDX1+PRDX2+PRDX3+PRDX5, PRDX1+PRDX2+PRDX4+PRDX5, PRDX1+PRDX3+PRDX4+PRDX5, oder PRDX2+PRDX3+PRDX4+PRDX5 ausgewählt sind.

5. Verwendung nach einem der Ansprüche 1-4, wobei alle fünf 2-Cys-PRDXs-Biomarker PRDX1+PRDX2+PRDX3+PRDX4+PRDX5 verwendet werden.

6. Ein Verfahren zur Diagnosestellung von Lupus-Nephritis (LN) und zur Differenzierung der Lupus-Nephritis (LN) von anderen chronischen Nierenerkrankungen (CKD), ausgewählt aus einer Gruppe, die aus IgA-Nephropathie (IgAN) und autosomal-dominanter polyzystischer Nierenerkrankung (ADPKD) besteht, bei einem Probanden, umfassend:
(a) einen Schritt der Identifizierung von mindestens zwei, drei, vier oder fünf 2-Cys-PRDXs-Biomarkern, die aus einer Gruppe von PRDX1, PRDX2, PRDX3, PRDX4 und PRDX5 ausgewählt sind in einer Serumprobe des genannten Probanden, und
(b) einen Schritt der Identifizierung von mindestens zwei oder drei oder vier oder fünf Biomarkern, die aus einer Gruppe von PRDX1, PRDX2, PRDX3, PRDX4 und PRDX5 ausgewählt sind, in einer Serumprobe eines gesunden Probanden und
(c) einen Schritt des Vergleichens der identifizierten Biomarker in einer Serumprobe des untersuchten Probanden mit den Markern, die in einer Serumprobe eines gesunden Probanden identifiziert wurden, wobei erhöhte Biomarker-Konzentrationen im Serum LN diagnostiziert werden und die LN von anderen CKD-Erkrankungen unterscheiden lassen, die aus der Gruppe ausgewählt sind, die aus IgA-Nephropathie (IgAN) und autosomal-dominante polyzystische Nierenerkrankung (ADPKD) besteht.

## Revendications

1. Utilisation d'au moins deux des 2-Cys-PRDX sériques choisies dans un groupe constitué de PRDX1, PRDX2, PRDX3, PRDX4 et PRDX5 comme biomarqueur sérique pour diagnostiquer la néphrite lupique (LN) et différencier la néphrite lupique (LN) d'autres maladies rénales chroniques (CKD) choisies dans le groupe constitué de la néphropathie à IgA (IgAN) et de la polykystose rénale autosomique dominante (ADPKD), dans lequel, lorsque les au moins deux biomarqueurs 2-Cys-PRDX comprennent une combinaison de PRDX1+PRDX4, la néphrite lupique (LN), la néphropathie à IgA (IgAN) et la polykystose rénale autosomique dominante (ADPKD) peuvent être différenciées davantage.

2. Utilisation selon la revendication 1, dans laquelle au moins deux biomarqueurs 2-Cys-PRDX sont choisis parmi une combinaison de PRDX1+PRDX2, PRDX1+PRDX3, PRDX1+PRDX4, PRDX1+PRDX5, PRDX2+PRDX3, PRDX2+PRDX4, PRDX2+PRDX5, PRDX3+PRDX4, PRDX3+PRDX5, ou PRDX4+PRDX5.

3. Utilisation selon la revendication 1 ou 2, dans laquelle au moins trois biomarqueurs 2-Cys-PRDX sont utilisés, choisis parmi une combinaison de PRDX1+PRDX2+PRDX3, PRDX1+PRDX2+PRDX4, PRDX1+PRDX3+PRDX4, PRDX1+PRDX2+PRDX5, PRDX1+PRDX3+PRDX4, PRDX1+PRDX3+PRDX5, PRDX1+PRDX4+PRDX5, PRDX2+PRDX3+PRDX4, PRDX2+PRDX3+PRDX5, PRDX2+PRD4+PRDX5, ou PRDX3+PRDX4+PRDX5.

4. Utilisation selon l'une des revendications 1 à 3, dans laquelle au moins quatre biomarqueurs 2-Cys-PRDX sont utilisés, choisis parmi une combinaison de PRDX1+PRDX2+PRDX3+PRDX4, PRDX1+PRDX2+PRDX3+PRDX5, PRDX1+PRDX2+PRDX4+PRDX5, PRDX1+PRDX2+PRDX3+PRDX5, PRDX1+PRDX2+PRDX4+PRDX5, PRDX1+PRDX3+PRDX4+PRDX5, ou PRDX2+PRDX3+PRDX4+PRDX5.

5. Utilisation selon l'une des revendications 1 à 4, dans laquelle les cinq biomarqueurs 2-Cys-PRDX PRDX1+ PRDX2+PRDX3+PRDX4+PRDX5 sont utilisés.

6. Méthode de diagnostic de la néphrite lupique (LN) et de différenciation de la néphrite lupique (LN) d'une autre maladie rénale chronique (MRC) choisie dans le groupe constitué de la néphropathie à IgA (IgAN) et de la polykystose rénale autosomique dominante (ADPKD) chez un sujet, comprenant
(a) une étape d'identification d'au moins deux, trois, quatre ou cinq biomarqueurs 2-Cys-PRDX choisis dans un groupe constitué de PRDX1, PRDX2, PRDX3, PRDX4 et PRDX5 dans un échantillon de sérum dudit sujet, et
(b) une étape d'identification d'au moins deux, trois, quatre ou cinq biomarqueurs choisis dans un groupe constitué de PRDX1, PRDX2, PRDX3, PRDX4 et PRDX5 dans un échantillon de sérum provenant d'un sujet sain, et
(c) une étape de comparaison des biomarqueurs identifiés dans un échantillon de sérum dudit sujet avec les marqueurs identifiés dans un échantillon de sérum d'un sujet sain, dans laquelle des concentrations élevées de biomarqueurs dans le sérum permettent de diagnostiquer une LN et de différencier la LN d'autres maladies liées à la CKD, choisies dans le groupe constitué par la néphropathie IgA (IgAN) et la polykystose rénale autosomique dominante (ADPKD).
